# EUROPEAN PATENT APPLICATION

(11) **EP 3 701 942 A1**
(43) Date of publication of application: **02.09.2020**
(21) Application number: 18871653.4
(22) Date of filing: 07.03.2018
(51) Int. Cl.: A61K 9/10, A61K 31/155, A61K 47/10, A61K 47/18, A61L 2/18, A61L 101/32, A61P 31/02

(54) **SOLUBILIZATION OF CHLORHEXIDINE BASE, ANITSEPTIC COMPOSITION AND DISINFECTANT COMPOSITION**

(30) Priority: 23.10.2017 RU 2017133879
(71) Applicant: The Limited Liability Company "Sun Systems", Moscow 119421 (RU)
(72) Inventor: KARDASH, Gennady Grigoryevich, Moscow 125475 (RU); ARTEMANN, Jean-Christian, 88500 Murcourt (FR); RYTSAREV, Alexander Yuryevich, Moscow 119421 (RU); HAPKINA, Elena Nikolaevna, Moscow 119330 (RU)
(74) Representative: Berggren Oy, Helsinki & Oulu
(86) International application number: PCT/RU2018/000146
(87) International publication number: WO 2019/083397

(57) **Abstract**

The present invention relates to a chlorhexidine base and to producing a solubilized form containing a chlorhexidine base amino acid in the presence of amine oxide. The invention relates to medicine and the pharmaceutical industry, particularly to novel antiseptic and disinfectant compositions that are active toward Mycobacterium tuberculosis, including strains that are multidrug resistant MDR, extensively drug resistant XDR, and pandrug resistant PDR; bacterial spores; and fungi; and said compositions having virucidal activity.

## Description

### Field of technology

The present invention relates to chlorhexidine base, preparation of a solubilized form containing chlorhexidine base and amino acid in the presence of amine oxide. It relates to medicine and the pharmaceutical industry, in particular to new antiseptic and disinfectant compositions active against Mycobacterium tuberculosis, including strains of polyresistant MDR, extremely resistant XDR and pan-resistant PDR, bacterial spores, fungi and having virucidal activity.

### Background

Chlorhexidine base belongs to the class of biguanides. Chemically, chlorhexidine base is 1, 1' - hexamethylene bis-[5-(4-chlorophenyl) - biguanide]. The formula of the chlorhexidine molecule C₂₂H₃₀CL₂N₁₀, in the form of a structural formula is presented below.

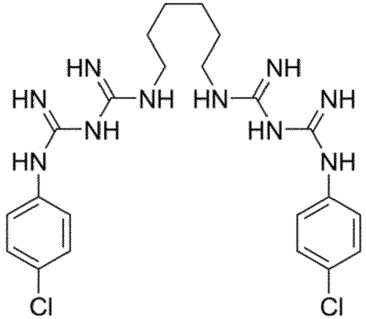

Chlorhexidine base is a white crystalline powder, practically insoluble in water and alcohol. Given the poor solubility of chlorhexidine base usually, chlorhexidine is used in the form of salts, mainly piazetta, bigluconate or dihydrochloride [Ruppert M., Schlagenhauf U. La clorhexidina en Odontologia. Aspectos generales. Quintessence (Ed. Espanola) 2005; 18:12-23; Franch M., Pascual A., Santos A. Colutorios en periodon-cia. Parte II. Arch Odontoestomatologia 2005; 21:471-87]. Bigluconate is the most soluble in water and alcohol, and therefore, this form is used in liquids for mouth rinses, gels and varnishes [Arevalo J.M., Arribas J.L., Calbo L., Hernandez M.J., Lizan M., Herruzco R. Guia del grupo de trabajo sobre desin-fectantes y antisepticos. Revision 1998. Medicina Preventiva 1998; 4:38-43.]. Moreover, this form has an additional advantage, since the physiological pH value released active ingredients with positive ion charge [Albertos J.M., Junquera L.M., Albertos M.T., Olay S., Lopez- Arranz E. La clorhexidina. Perspectiva actual. Ann Odontoestomatologia 1996; 5:217-23.]. Chlorhexidine acts at the level of the cell membrane, increasing its permeability [Hugo W.B. Disinfection mechanisms. In: Russell A.D., Hugo W.B., Ayliffe G.A.J., eds. Principles and Practice of Disinfection, Preservation and Sterilization. Oxford: Blackwell; 1992:187-210].
First stage of chlorhexidine action is its fast adsorption on microbial wall, due to the presence of two main and symmetrical groups of chlorophenylguanide attached to the lipophilic chain of hexamethylene, which form a dicationic molecule that interacts with bacterial surface [Musteata F.M., Pawliszyn J. Assay of stability, free and total concentration of chlorhexidine in saliva by solid phase microextraction. J Pharm Biomedical Analysis 2005; 37:1015-24]. Conditions for their binding are most favorable in a neutral or slightly alkaline pH; amount of chlorhexidine adsorbed depends on concentration of the agent. The outer layer of bacterial cell wall carries a negative charge, which usually stabilizes in the presence of cations such as Mg2 + and Ca2 +; this is the basis of action of most cationic antiseptics, including chlorhexidine, which have high affinity for bacterial cell wall [Gilbert P., Moore L.E. Cationic antiseptics: diversity of action under a common epithet. J Appl Microbiol 2005; 99:703-15.
Salts of chlorhexidine, in particular bigluconate is an antiseptic with bactericidal and fungicidal effect. Although it is not considered a viricide, some slight activity was noted in relation to lipid membranes of viruses, such as HIV, herpes 1 and 2, influenza A [Bemimoulin J.P. Recent concepts in plaque formation. J Clin Periodontology 2003; 30:7-9.]. Chlorhexidine bigluconate can inhibit growth of spore and exerts bacteriostatic action against certain bacteria, but has no effect on acid-resistant bacteria [Junco-Lafuente M.P, Baca-Garcia P., Mesa-Aguado F.L. Utilizacion de la clorhexidina en la prevencion oral de pacientes de la tercera edad. Revista del Ilustre Consejo General de Colegios de Odontologos y Estomatologos de Espana 2001; 6:81-9].
In low concentrations Chlorhexidine bigluconate is bacteriostatic agent, however, when it is removed from the environment, the functions of bacterial cells recover [Fardal O., Turnbull R.S. A review of the literature on use of chlorhexidine in dentistry. J American Dental Association 1986; 112:863-9].
As antiseptics and disinfectants chlorhexidine salts have found their application in a variety of areas.
Dozens of aseptic and disinfectant compositions with chlorhexidine salts are registered and produced. As an active component of chlorhexidine bigluconate is part of the drug Citeal (chlorhexidine bigluconate along with hexamidine and chlorocresol), produced by the French firm "PIERRE-FABRE MED." and in the composition of the drug "Plivasept", produced by the Croatian company "PLIVA", in the form of emulsions containing chlorhexidine gluconate. 7 patents are devoted to preparation and application of chlorhexidine derivatives and compositions based on it [(patent WO 95/12395-firm "Calgon Vestal Lab" (USA); patent EP 306455 - firm "Warner Lambert Co" (USA); patent WO 93/9770 - firm "Pierre Fabre"(France); patent EP 200607 - firm "P. F. Cosmetique "(France) and others] Firm" Ivoclar AG " (Germany) suggested use of chlorhexidine with HF in toothpastes (patents de 415397, EP 539811, US 5393516).

Phenyl derivatives and cyan derivatives of chlorhexidine have been considered as potential highly active fungicides. Firm "Bayer" (Germany.) developed on the basis of a composition of derivatives of phenyl-guanidine-imidazole and tetrahydropyrimidine (patent EP 279343) anthelmintic agent. BASF (Germany) (patents).2812945,3922232), firm "American Cyanamid Co" (USA) (patents EP 406699, EP534501, US 5449809), firm "Coro Jokko K. K." (Japan.) (jap patents. 61-25706, 61-027), the company " Hakko Chemical "(India). (us patents 5116838) developed new biocides. However, their industrial prospects are very small due to the high environmental requirements and difficulties of disposal of phenolic and cyanide-containing waste.
The biocidal properties of chlorhexidine can be enhanced by the introduction of iodine (patent EP 473320) or eugenol (patent WP 93/9770) compounds into the composition.

Promising group of biocidal drugs based on guanidine-polyhexamethylenbiguanidine and its derivatives are presented in Russian patents No. 2351365 "Antiseptic composition "CHLOR-DIX", which contains a combination of known, publicly available and inexpensive antiseptics bigluconate chlorhexidine and dioxidine, RU No. 2147032 "Bactericidal-detergent", in which the main active substance is chlorhexidine bigluconate or chlorhexidine gluconate, or chlorhexidine salt. Activity at action on acid-resistant forms of bacteria such as Helicobacter pylori and Micobacterium tuberculosis was noted in a composition according to patent No. 2351365.
However, the description does not specify what types of strains of Micobacterium tuberculosis with what degree of resistance participated in the study. We can assume, that there were used strains sensitive to antibacterial drugs. All known and described products based on chlorhexidine salts have a wide range of active, quick and strong bactericidal effects on gram-positive and gram-negative bacteria, which have spermicidal activity, activity against acid-resistant forms of bacteria. Each known preparation containing chlorhexidine salt, is enhanced by one or another substance to improve consumer quality, for example alkyldimethylbenzylammonium chloride (katamine AB) or didecyldimethylammonium chloride, non-ionic surface-active substance, quaternary ammonium salts, water-soluble oxyethylated monoalkyl phenols based on propylene trimers, etc., acts against certain types of bacteria, but ineffective against resistant Mycobacterium tuberculosis, bacterial spores, fungi and have low virucidal activity.
Given that many antiseptic and disinfectant compositions have already developed resistance from microorganisms, the mechanism of action of chlorhexidine on the microbial cell and the potential of chlorhexidine base allow us to look for ways to obtain other forms of chlorhexidine base, soluble in water and alcohol.
One ways is obtain chlorhexidine base solubilizers.
In the report "Associations based on chlorhexidine and a synergistic effect", G. Kardash et al. // Materials of the VIII All-Russian Congress of Epidemiologists, Microbiologists and Parasitologists: v. 4. Moscow. Rosineks, 2002. - P. 21-22, was outlined an approach to conversion of chlorhexidine base to a soluble form and to synergistic increase of biocidal activity by formation of chlorhexidine base and glycine associates. The report presents comparative data on the biocidal activity of chlorhexidine base and glycine associates, shows a synergistic effect on test cultures of Gr + (Staphylococcus aureus), Gr + (Klebsiella pneumonia) and Pseudomonas aeruginosa. However, the associate of chlorhexidine base and glycine quickly loses stability. The approach to the preparation of a chlorhexidine base solubilized form is described in the report "Solubilization of chlorhexidine in aqueous micellar solutions of nonionic surfactants," // Abstracts of the XXIII All-Russian Conference "Structure and Dynamics of Molecular Systems", Moscow, 2016. - P. 74-74.
In this work, the methods of immobilization of chlorhexidine with micelles of nonionic surfactants were studied by UV spectroscopy, refractometry, and dynamic light scattering. It was shown that the equilibrium solubility of chlorhexidine in aqueous micellar solutions increases linearly with increasing concentration of nonionic surfactants. The solubilization capacity of micelles toward chlorhexidine was determined, which characterizes the ratio of the number of chlorhexidine molecules and nonionic surfactants in micelles. With an increase in the number of hydroxyethyl units in the nonionic surfactant molecule, the values of the solubilization capacity increase in the order: Lutensol XL-79, Neonol 9-10, Tween 80. The standard Gibbs energy of solubilization was calculated, and the effect of solubilized chlorhexidine on the micelle sizes of nonionic surfactants was calculated. Chlorhexidine was found to be localized in the oxyethyl layer of micelles, and not in the hydrocarbon core. The pH values in all cases were in the range of 6-8, which is optimal for the manifestation of the maximum bactericidal activity of chlorhexidine. However, it has not been proven that the solubilized chlorhexidine base, obtained using nonionic surfactants, actually showed maximum bactericidal and other types of activity.

However, the actual bactericidal activity of the obtained solubilized forms of chlorhexidine base was not shown.

### Summary of the invention

The purpose of the invention - creation of a well-soluble solubilizate of chlorhexidine base, creation of antiseptic and disinfectant compositions on the basis of solubilized form of chlorhexidine base, which is active against Mycobacterium tuberculosis including strains that are multidrug resistant MDR, extremely resistant XDR and pan-resistant PDR, bacterial spores, fungi and has virucidal activity, high safety and affordability.
**In the first aspect of the invention**, it was discovered that associates of chlorhexidine base and amino acid (alanine, glutamic acid, glutamine, lysine, arginine, histidine, cysteine, methionine, glycine, aspartic acid, asparagine, etc.) in the presence of amine oxide form a morphological structure - solubilizates of micellar and lamellar type, which is well dissolved in water and alcohol.
It was found that the chlorhexidine base and amino acid solubilized form in the presence of amine oxide constitute smooth lamellar formations of the mycelial and lamellar type - liquid crystals.
The data from UV and NMR spectroscopy, EPR showed that during solubilization process in lamellar micelles chlorhexidine base enters inside micelles, localising between the hydrocarbon ends and, thereby, pushes apart the layers of the molecular chains, forms dendritic or liquid crystals. A new morphological structure of liquid crystals is formed, which differs from the morphology of the initial components of chlorhexidine base and amino acide (glycine). The appearence of a new crystal modification is preceded by an ordered state of molecules in a solution with electrostatic and hydrogen intermolecular bonds, which causes the appearance of an abnormally high viscosity at a low concentration of components (0.1-1%).
New morphological structure showed synergistic biocidal activity against microorganisms, including mycobacterium tuberculosis of the multi-resistant MDR strain, the extremely resistant XDR and against the pan-resistant PDR strain, bacterial spores, fungi and viruses.
To understand the mechanism of synergistic effect, studies were carried out using various physicochemical methods that showed the protonation of the imino group of the chlorhexidine molecule and the presence of a new morphological structure of liquid crystals in dilute solutions of chlorhexidine base and amino acide (glycine).
The IR spectrum of solid samples of chlorhexidine base, chlorhexidine-glycine solubilizate, amine oxide was obtained on an M-40 instrument (Germany) in the form of powders pressed with EHF.

Analyzing the IR spectrum, it can be argued that carboxyl groups of glycine are in charged form -COO⁻.- In the field of absorption of -COOH group at 1700 cm⁻¹ bands are not found in the spectrum of glycine, nor in the spectrum of the mixture of glycine with chlorhexidine base. At the same time, in the field of COO absorption groups at 1600 cm⁻¹ and 1400 cm⁻¹ there are strong bands on both spectrum.
These data prove the mechanism of dissolution of chlorhexidine bases solubilizate in aqueous solutions, which occurs by protonation of chlorhexidine base.
UV spectrum of chlorhexidine solubilizate obtained by UV spectrophotometer M-80 (Germany) confirmed the conclusion of IR spectroscopy data on the cause of dissolution of chlorhexidine base in an amino acid (glycine) solution in the presence of amine oxide, which is due to the transfer of proton from the amino acid (glycine) to chlorhexidine base.

**In the second aspect of the invention**, antiseptic and disinfectant compositions were obtained using solubilizate of chlorhexidine base and auxiliary substances, that are active against tuberculosis mycobacteria, including the multi-resistant MDR strain, the extremely resistant XDR and the pan-resistant PDR.

One of the models of antiseptic composition in the form of concentrate contains chlorhexidine base, amino acid, amine oxide, excipients, in the following ratio, wt.%:
Chlorhexidine base-0,1- 1,0 %
Amine oxide-0.2 - 5.0 %
Amino acid-0,05-2,0 %
Glycerin-0,0 - 5,0 %
Dye-0.01-0.1 %
Water - up to 100 %
As amino acid are used leucine, tyrosine, therin, glutamine, asparagine, phenylalanine, alanine, lysine, argenine, histidine, glycine, cysteine, valine, proline, methionine, threonine, hydroxylysine.
As amine oxide are used N-lauryl-N, N-dimethylaminoxide (Barlox-12), catalog of the company "Lonza".
As a dye are used anthraquinone dye, karmuazin (E122) or triarylmethane dye (E133).
Antiseptic composition according to the invention has antibacterial action, including action against Mycobacterium tuberculosis, fungicidal, virucidal and sporicidal activity, and is intended for aseptic hand treatments, pre-sterilization of medical devices (including flexible endoscopes and instruments) and also for disinfection of objects of patients care, hygiene of medical staff and household purposes, does not cause corrosion of metal products.

### Brief description of the drawings

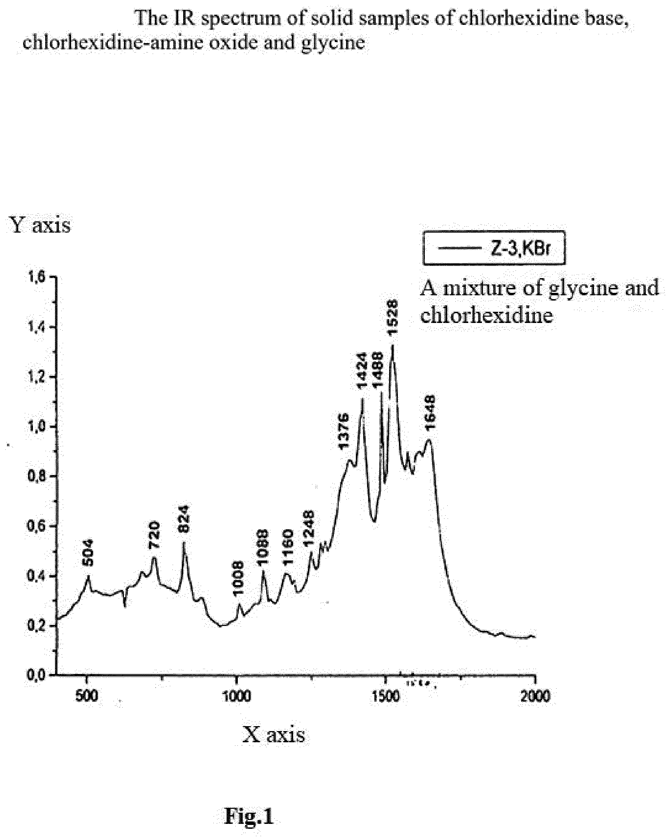

Fig. 1. IR spectrum of solid samples of chlorhexidine base, chlorhexidine-glycine solubilizate and amine oxide.

### Detailed Description of the invention

**Example 1**. To obtain 100 ml of aseptic composition in the form of a concentrate at room temperature 1 ml amine oxide Barlox-12 , catalog of the company Lonza , dissolved in 100 ml of water, add 0.15 g of chlorhexidine base, of 0.05 g glycine, 0.01 g of the dye E133. The obtained composition with pH 7.5-8 is an aseptic concentrate.

**Example 2**. Carried out similarly to example 1, but the composition includes 0.5 g chlorhexidine base, 3.5 ml Barlox 12, 1.5 g alanine, 2 ml glycerol, carmoisine (E122) 0.05 g.

**Example 3**. Carried out similarly to example 1, but the composiyion includes 1 g chlorhexidine base 5 ml Barlox 12, glutamine 2.0 g, 2 ml glycerol, anthraquinone dye 0.05 g.

A series of experiments carried out to confirmed high activity of antiseptic composition against *Micobacterium tuberculosis,* MDR poly-resistant strain and extremely resistant XDR, which are not sensitive to the 1st, 2nd and 3rd classes of antibacterial drugs and to pan-resistant PDR, which is not sensitive to all known antibacterial drugs of all classes.

Laboratory strain of *Micobacterium tuberculosis* H37Rv and clinical (wild) MDR, XDR, and PDR strains isolated from the diagnostic material of patients with pulmonary tuberculosis treated in a hospital of the Central Research Institute of tuberculosis of the Russian Academy of medical Sciences (RAMS) were used as biotests.

Two clinical strains are characterized taking into account effect of anti-TB drugs on them as sensitive and marked FEEL-1 and FEEL-2. Three others with varying degrees of resistance are marked MDR, XDR and PDR strain.

In accordance with the work plan culture of selected strains of mycobacteria tuberculosis was grown for 21 days in a dense nutrient medium Levenstein-Jensen (international standard). From the grown culture was prepared a suspension of bacteria with an initial concentration of 5 · 10⁸, i.e. containing 500 million microbial bodies in 1 ml of solution. Test substance was already-to-use solution. The exposure time to the culture of mycobacterium tuberculosis was 30 minutes and 60 minutes.

The experiment was carried out using a modified immersion method. The ratio of the suspension of Mycobacterium tuberculosis and the aseptic composition according to Example 2 (diluted in a ratio of 1 ml of concentrate per 100 ml of water) was 1: 1, i.e. 2 ml of a suspension of Mycobacterium tuberculosis of various strains and 2 ml of a solution of an aseptic composition concentrate were poured into test tubes. Thus, the concentration of bacterial suspension used in the work was 5 · 10⁴. This concentration of microbial bodies in the suspension corresponds to the required number of mycobacteria to detect them in the diagnostic material at stained smears by the method of Ziehl-Nielsen (light microscopy). After a given exposure, the mixture was centrifuged for 15 minutes. at 3000 rpm. The resulting precipitate was washed twice with distilled water. The inoculum dose was 0.5 ml of sediment per test tube with Finn-2 solid nutrient medium and 0.5 ml of sediment per test tube with Middlebrook 7N9 liquid nutrient medium. Control tubes were used, in which a suspension of mycobacteria of the test strains used was seeded and which was not subjected to treatment with the substance of the composition. Crops on dense media were incubated in a thermostat at a temperature of 37 0C; crops on liquid media - in an automated system Bactec MGIT-960. Results on dense nutrient medium were collected on 28, 42 and 70 days from the time of sowing. The results on the liquid nutrient medium were recorded on the Bartec MGIT-960 apparatus.

Table 1 shows the results of the cultural research antibacterial composition. Microbiological study of the compositions for bactericidal activity against Mycobacterium tuberculosis, including in relation to the polyresistant strain of MDR and extreme resistant XDR, not sensitive to 1st, 2nd and 3rd classes of antibacterial drugs and in relation to pan-resistant PDR, not sensitive to all known antibacterial drugs of all classes, in the Central Research Institute of tuberculosis of the Russian Academy of medical Sciences (RAMS) according to normative and technical documentation, order of the Ministry of health of the Russian Federation No. 109 dd. 21.03.2003, Annex Nº 1, Sanitary rules SP 1.2.731-99.

**Table No. 1 Results of a cultural study of the effect of antiseptic composition containing chlorhexidine base**

| Strains *Mycobacterium tuberculosis* | antiseptic composition containing chlorhexidine base | | | | | |
|---|---|---|---|---|---|---|
| | Dense medium 70 days' | | Control | The control Liquid medium 42 days | | Control |
| | 30 min | 60 min | | 30 min | 60 min | |
| H37RV-laboratory | - | - | >100 | - | - | >100 |
| FEEL-1 Clinical | | | >100 | | | >100 |
| FEEL-2 Clinical | | | >100 | | | >100 |
| MDR-strains - Clinical | - | - | >100 | - | - | >100 |
| XDR-strains - Clinical | - | - | >100 | - | - | >100 |
| PDR-strains - Clinical | - | - | >100 | - | - | >100 |

| | | | | | | |
|---|---|---|---|---|---|---|
| Where: (>100) - growth of colonies of Mycobacterium tuberculosis on a dense and liquid nutrient medium; (-) - no growth of Mycobactérium tuberculosis on dense and liquid nutrient Nutrient medium; Results of conducted researches showed high and stable activity in respect of the virulent clinical strains with Mycobacterium tuberculosis with different degree of resistance. On day 70 or more from the moment of sowing, no growth of colonies of polyresistant MDR strain, extremely resistant XDR and pan-resistant PDR was detected. | | | | | | |

### Options for the implementation of the invention

Versions of the disinfectant concentrate compositions containing Chlorhexidine base, aminoxide, Quaternary ammonium compound (QAC), acidity regulator, anti-corrosion additive, thickener, auxiliary substances, at the following component ratio, mass fraction %:

**Option 1**

| | |
|---|---|
| Chlorhexidine base is | -1.0 - 2.0 % |
| Aminoxide | -1,0 - 15,0 % |
| Quaternary ammonium compound (QAC) | - 5,0 -10,0 % |
| Acidity regulator | -0,1- 1,0 % |
| Anti-corrosion additive | - 0,1 - 1,0 % |
| Dye | -0.01-0.1 % |
| Water | - up to 100 % |

It is preferable that as QAC is used alkyldimethylbenzylammoniumchloride (Katamin A or B). Preferably, triethanolamine, Trilon A, B or C are used as an anticorrosive additive. Preferably, malic acid is used as an acidity regulator. It is preferable that the dye used is dye E133.

**Option 2** - concentrate disinfectant composition contains chlorhexidine base, amino acid, amine oxide, excipients, at the following ratio of components, mass fraction %:

| | |
|---|---|
| Chlorhexidine base | -0,5 - 5,0 % |
| Aminoxide | -5,0 - 30,0 % |
| Polyethylene glycol | -0,2 - 3,0 % |
| Amino acid is | -0,0- 2,0 % |
| Dye | -0.01-0.1 % |
| Water | - up to 100 % |

As an amino acid used leucine, tyrosine, terine, glutamine, asparagine, phenylalanine, alanine, lysine, argenine, histidine, glycine, cysteine, valine, proline, methionine, threonine, hydroxylysine.

As amine oxide use N-lauryl-N, N-dimethylamine (Barlox-12), catalogue of the company Lonza.

Triarylmethane dye (E133) is used as a dye.

**Option 3**-concentrate disinfectant composition contains chlorhexidine base, amino acid, amine oxide, excipients, at the following component ratio, mass fraction %:

| | |
|---|---|
| Chlorhexidine base is | - 0.5 - 65,0 % |
| Amino acid | -0,1-2,0% |
| Amine oxide | -0.2 - 5.0 % |
| Dye | -0.01-0.1 % |
| Alcohol | - 10,0 -30,0 % |

As an amino acid used leucine, tyrosine, terine, glutamine, asparagine, phenylalanine, alanine, lysine, argenine, histidine, glycine, cysteine, valine, proline, methionine, threonine, hydroxylysine.

As aminoxide use N-lauryl-N, N-dimethylamine (Barlox-12), catalogue of the company Lonza. Triarylmethane dye (E133) is used as a dye.

Ethanol, propanol, methanol are used as alcohol.

Preparation of concentrates of disinfectant compositions of Variants 1, 2 and 3 is carried out similarly to example 1, by mixing the components at room temperature in the specified proportions of active substances.

### Industrial application

To investigate the disinfectant activity against Mycobactérium tuberculosis strains, gram-negative and gram-positive bacteria, fungi, concentrates of disinfectant compositions according to Variants 1, 2 and 3 were diluted in water in a ratio of 1:100.

Often found in surgical practice bacterial agents such as Enterobacter spp., Citobacter spp. Klebsiella, Escherichia coli, Pseudomonas aeruginosa, Staphylococcus aureus, Poliomyelitis, Candida were sown on nutrient media by generally accepted methods and evaluated in accordance with the requirements of Normative documentation(ND) "Methods of laboratory researches and tests of disinfectants for an assessment of their efficiency and safety" (P 4.2.2643-10) and ND " Normative indicators of safety and effectiveness of disinfectants, subject to control during the mandatory certification", Nº 01-12 / 75-97.

The study was conducted in the Scientific Research Institute of Disinfectology of Rospotrebnadzor of RF.

Data of disinfecting activity are given in Table 2.

**Table No. 2**

| Versions of disinfection compositions (solution 1%) | Efficiency of test objects, contaminated test microorganisms (in % to 100% hygienic normative) | | | | | | |
|---|---|---|---|---|---|---|---|
| | M. Terrae strain DSM/ exposure time | Candida Albicans (strain1 5)/ exposure time | Staphylococcus aureus (strain 906)/ exposure time | Microsporum gypseum/ exposure time | Escherichia coli/ exposure time | Bacillus cereus (strain 96)/ exposure time | Poliomyel itis)/ exposure time |
| Antiseptic composition | 100%/ 30 min | 100%/ 30 sec | 100%/ 30 sec | 100%/ 1 min | 100%/ 30 sec | 100% / 3 min | 100% / 30 min |
| 1 | 100% / 60 min. | 100%/ 30 sec | 100% / 30 sec | 100%/ 1 min | 100% / 30 sec | 100%/ 5 min | 100% / 60 min |
| 2 | 100%/ 30 min. | 100% / 45 sec | 100%/ 30 sec | 100%/ 1 min | 100% / 30 sec | 100%/ 3 min | 100% / 30 min |
| 3 | 100% / 60 min. | 100% / 45 sec | 100%/ 30 sec | 100%/ 1 min | 100% / 30 sec | 100%/ 3 min | 100% / 60 min |

Thus, the inventive antiseptic composition possesses unique bactericidal activity against Mycobactérium tuberculosis, including multidrug-resistant strain of MDR, extreme drug-resistant XDR which is not sensitive to 1st, 2nd and 3rd classes of antibiotics and pan-resistant PDR which is not sensitive to all known antibiotics of all classes.

Variants of disinfectant compositions have increased qualities-the ability to affect a wide range of bacteria - M. Terrae, Enterobacter spp., Citobacter spp., Klebsiella, Escherichia coli, Pseudomonas aeruginosa, Staphylococcus aureus, Poliomyelitis, Candida, have a low cost, due to the use of chlorhexidine base, which is a semi-product in the production of salts and derivatives of this compound and ease of preparation.

## Claims

1. A method of solubilization of chlorhexidine base consisting in the micellar and lamellar microencapsulation of chlorhexidine base in the presence of amino acids and amine oxide.

2. Solubilization of the chlorhexidine base according to claim 1, N-lauryl-M, N-dimethylamine oxide is used as amine oxide, leucine, tyrosine, terine, glutamine, asparagine, phenylalanine, alanine, lysine, argenine, histidine, glycine, cysteine, valine, proline, methionine, threonine, hydroxylysine are used as amino acid.

3. An antiseptic composition in the form of a concentrate containing chlorhexidine base, amino acid, amine oxide, glycerin, dye and water in the following ratio, mass fraction %: Chlorhexidine base - 0.1-1.0%
| | |
|---|---|
| Amine oxide | - 0.2 - 5.0% |
| Amino acid | - 0.05-2.0% |
| Glycerin | - 0.0 - 5.0% |
| Dye | - 0.01 - 0.1% |
| Water | - up to 100%: |

4. The antiseptic composition according to claim 3, wherein N-lauryl-N, N-dimethylamine oxide is used as amine oxide, leucine, tyrosine, terine, glutamine, asparagine, phenylalanine, alanine, lysine, argenine, histidine, glycine are used, cysteine, valine, proline, methionine, threonine, hydroxylysine, anthraquinone dye, cannoisine (E122) or triarylmethane dye (E133) are used as a dye.

5. The antiseptic composition according to claim 3 is intended for therapeutic and non-therapeutic use against strains of multi-resistant MDR, extremely resistant XDR and pan- resistant PDR against *Micobacterium tuberculosis.*

6. A variant of a concentrate of a disinfecting composition containing chlorhexidine base, amine oxide, quaternary ammonium compound, acidity regulator, anti-corrosion additive, dye and water in the following ratio, mass fraction %:
Chlorhexidine base -1,0 - 2.0%
Amine oxide -1.0 - 15.0%
Quaternary ammonium compound - 5.0 -10.0%
Acidity regulator - 0.1 - 1.0%
Corrosion inhibitor - 0.1 - 1.0%
Dye - 0.01 -0.1%
Water - up to 100%

7. A variant of a concentrate of a disinfecting composition containing chlorhexidine base, amino acid, amine oxide, polyethylene glycol, dye and water in the following ratio, mass fraction %:
Chlorhexidine base - 0.5 - 5.0%
Amine oxide - 5.0 - 30.0%
Polyethylene glycol - 0.2 - 3.0%
Amino acid - 0.0-2.0%
Dye - 0.01 -0.1%
Water - up to 100%

8. A variant of a concentrate of a disinfecting composition containing chlorhexidine base, amino acid, amine oxide, dye and alcohol in the following ratio, mass fraction %:
Chlorhexidine base - 0.5 - 65.0%
Amino acid - 0.1-2.0%
Amine oxide - 0.2 - 5.0%
Dye - 0.01 - 0.1%
Alcohol - 10.0 -30.0%

9. The concentrate of the disinfectant composition according to claims 6, 7 and 8, where M-lauryl-N, N-dimethylamine oxide of the quaternary ammonium compound alkyl dimethylbenzylammonium chloride (Catamine A or B) is used as amine oxide, triethanolamine, Trilon A, B are used as anti-corrosion additives, malic acid is used as acidity regulator, the dye E 133 is used as a dye.
